# EUROPEAN PATENT APPLICATION

(11) **EP 4 566 595 A1**
(43) Date of publication of application: **11.06.2025**
(21) Application number: 23214884.1
(22) Date of filing: 07.12.2023
(51) Int. Cl.: A61K 31/164, A61K 9/00, A61K 31/56, A61K 31/58, A61P 11/02, A61P 37/08

(54) **COMBINATION PREPARATION FOR THE TREATMENT OF ACUTE AND CHRONIC RHINOSINUSITIS**

(71) Applicant: Roth, Bernhard, 97074 Würzburg (DE)
(72) Inventor: Roth, Bernhard, 97074 Würzburg (DE)
(74) Representative: Uexküll & Stolberg

(57) **Abstract**

The present application provides a novel combination preparation for us in the treatment of acute, chronic and allergic rhinosinusitis which does not cause dry nasal mucosa and is nevertheless very effective in the treatment of the above diseases. The combination preparation comprises an active ingredient selected from mometasone and/or fluticasone or a pharmaceutically acceptable salt or solvate of any one of the foregoing, in combination with dexpanthenol or a pharmaceutically acceptable salt or solvate thereof, as well as a pharmaceutically acceptable excipient

## Description

### FIELD OF INVENTION

The resent invention relates to a combination preparation for the treatment of acute and chronic rhinosinusitis.

### BACKGROUND OF INVENTION

Chronic rhinosinusitis, including nasal polyps, is an inflammatory disease of the nose and sinuses. As disclosed in S.H. Cho et al. "Medical Management Strategies in Acute and Chronic Rhinosinusitis", J. Allergy Clin. Immunol. Pract., 2020, 1159-1564 (doi: 10.1016/j.jaip.2020.02.020), chronic rhinosinusitis, historically, has been considered to be caused by upper airway anatomical abnormalities. However, today that concept has changed, for it is now recognized as an inflammatory disorder of the nasal and sinus mucosa. Acute rhinosinusitis is usually caused by a viral infection, whereas chronic rhinosinusitis is a persistent and heterogeneous inflammatory disorder.

Common treatments of acute, chronic and also allergic rhinosinusitis include treatment with topical intranasal or oral corticosteroids. Long-term treatment with corticosteroid nasal spray reduces inflammation and nasal polyp size, and improves nasal symptoms such as nasal blockage, rhinorrea, and the loss of smell. Corticosteroid intranasal drops may be used when intranasal spray fails to demonstrate efficacy. Short courses of oral steroids are recommended in severe chronic rhinosinusitis with nasal polyps or when a rapid symptomatic improvement is needed. The mechanism of action of nasal corticosteroids involves the drug molecule binding the cytoplasmic CS receptor (CSR) which, after being transported into the nucleus, binds the GRE sequence which can be found in numerous gene promoters, thus affecting the expression of respective genes.

The corticosteroid budesonid, as an aqueous nasal spray, has been shown to have a positive impact for those with chronic rhinosinusitis and perennial allergic rhinitis. However, budesonid and other topical corticosteroids, that were initially launched on the market, led to the absorption of considerable amounts of the active ingredient in the bloodstream. This was likely the cause for a number of systemic side effects. The corticosteroids mometasone and fluticasone have also been suggested for the treatment of acute and chronic rhinosinusitis and show the effect of absorption into the bloodstream to a considerably lesser degree; less than 2% of the applied active ingredient is absorbed in the bloodstream. Thus, they have been preferably applied as a nasal spray for the treatment of acute and chronic rhinosinusitis.

Fluticasone is a manufactured glucocorticoid used to treat nasal symptoms. Both the esters, fluticasone propionate and fluticasone furoate, are also used as topical anti-inflammatories and inhaled corticosteroids.

Fluticasone propionate, sold under the brand names Flovent and Flonase among others, is described in GB 2 088 877. It is a corticosteroid known to exhibit topical anti-inflammatory activity. Fluticasone propionate is used by powder or aerosol inhalation for the prophylaxis of asthma. The nasal spray is used for prevention and treatment of allergic rhinitis.

Fluticasone furoate, sold under the brand name Flonase Sensimist among others, is a corticosteroid for the treatment of non-allergic and allergic rhinitis administered by a nasal spray. It has been found to be effective to administer fluticasone propionate in the form of aerosols containing small particles of the medicament, conventionally prepared by micronization. Conventionally, fluticasone propionate aerosols have been administered by means of metered dose inhalers, which are designed to deliver a fixed unit dosage of medicament per actuation.

Generally, administration forms of fluticasone are designed so that each actuation of fluticasone delivers 50 µg of fluticasone propionate or fluticasone furoate in 100 mg of formulation through a nasal adapter. The recommended starting dosage of fluticasone propionate or fluticasone furoate in adults is 2 sprays (50 µg of fluticasone propionate each) in each nostril once daily (total daily dose, 200 µg).

Mometasone furoate is administered likewise in doses of 50 µg of mometasone furoate in 100 mg of formulation through a nasal adapter. Mometasone furoate is described in EP 0 057 401.

Generally, it takes days and weeks of topical corticosteroids to build up their strength, thus normally requiring the drug to be administered for a longer period of time. Topical corticosteroids therefor include preservatives. The anti-secretory effect, as well as the preservatives added often cause dry nasal mucosa to a greater or lesser extent. Acute or chronic inflammations cannot heal due to the dry mucous membranes which delays healing and fastens new infections. The phenomenon of dry mucous membranes also occurs when using nasal sprays with vasoconstrictor compounds to reduce swelling.

It is therefore an object of the present invention to provide a combination preparation for the treatment of acute and chronic rhinosinusitis containing mometasone and/or fluticasone useful for topical administration which does not cause dry nasal mucosa.

### SUMMARY OF THE INVENTION

The object is solved with a combination preparation as claimed in claim 1. Preferred embodiments are described in the appending claims.

In an embodiment, the present invention provides a combination preparation for the treatment of acute, chronic and allergic rhinosinusitis in the form of a nasal spray or nasal droplets comprising
(a) mometasone and/or fluticasone or a pharmaceutically acceptable salt or solvate of any one of the foregoing, and
(b) dexpanthenol or a pharmaceutically acceptable salt or solvate thereof as active pharmaceutical ingredients, as well as a pharmaceutically acceptable excipient.

It has shown that the combination preparation as described is effective in reducing the incidence of dry nasal mucosa and side effects associated therewith even after topical treatment thereof over an extended period of time. Furthermore, it has surprisingly shown that a faster onset of the pharmaceutical effect is achieved with the combination preparation according to the present invention than with mometasone and/or fluticasone alone.

Fluticasone and/or mometasone are preferably be administered in the usual form, administering 40 µg to 60 µg, preferably about 50 µg of fluticasone propionate or fluticasone furoate or mometasone furoate in 100 mg of formulation as a spray.

Dexpanthenol is the alcoholic analogue of pantothenic acid, and it is oxidized to this substance within the tissues.

It can be administered as nasal drops in concentrations ranging from 5 mg/mL to 40 mg/mL, preferably between 5 mg/mL and 20 mg/mL.

It has shown that the combination preparation is particularly effective when mometasone and/or fluticasone or a pharmaceutically acceptable salt or solvate of any one of the foregoing in concentrations raging from 0.02 mg/0.1 mL to 0.1 mg/0.1 mL is combined with 2-10 % w/v dexapanthenol.

Topical corticosteroids and in particular mometasone and fluticasone are hydrophobic, whereas dexpanthenol is hydrophilic. Therefore, the active agents cannot be combined directly. In an embodiment, the present invention therefore provides the combination preparation in form of a suspension, preferably in the form of an aqueous suspension, where dexapanthenol or a pharmaceutically acceptable salt thereof is present in the aqueous phase and mometasone and/or fluticasone or a pharmaceutically acceptable salt on any one of the foregoing being suspended therein.

Preferably fluticasone is administered in the form of fluticasone proprionate.

In an embodiment, the present application also relates to a suspension formulation suitable for nasal administration comprising (a) mometasone and/or fluticasone or a pharmaceutically acceptable salt or solvate of any one of the foregoing, and (b) dexpanthenol or a pharmaceutically acceptable salt or solvate thereof as active pharmaceutical ingredients.

In some cases, however, it is preferable that one or more of the ingredients of the combination preparation according to the present invention are present in microencapsulated form. In this form the hydrophilic and hydrophobic properties of the active ingredients addressed above can be combined in one medication.

## Claims

1. A combination preparation for us in the treatment of acute, chronic and allergic rhinosinusitis comprising an active ingredient selected from mometasone and/or fluticasone or a pharmaceutically acceptable salt or solvate of any one of the foregoing, in combination with dexpanthenol or a pharmaceutically acceptable salt or solvate thereof, as well as a pharmaceutically acceptable excipient.

2. The combination preparation of claim in the form of a nasal spray or nasal droplets comprising
(a) mometasone and/or fluticasone or a pharmaceutically acceptable salt or solvate of any one of the foregoing, and
(b) dexpanthenol or a pharmaceutically acceptable salt or solvate thereof as active pharmaceutical ingredients, as well as a pharmaceutically acceptable excipient.

3. The combination preparation of claim 2, which is present in the form of nasal drops wherein fluticasone and/or mometasone or a pharmaceutically acceptable salt or solvate of any one of the foregoing are contained in a solution or suspension in concentrations ranging from 0.02 mg/0.1 mL to 0.1 mg/0.1 mL combined with 2-10 % w/v dexapanthenol.

4. The combination preparation of any one of claims 2 or 3, which is present in the form nasal drops wherein fluticasone and/or mometasone or a pharmaceutically acceptable salt or solvate of any one of the foregoing are contained in a solution or suspension in concentrations ranging from 0.05 mg/0.1 mLto 0.1 mg/0.1 mL combined with 5-10 % w/v dexapanthenol.

5. The combination preparation of any one of the foregoing claims wherein dexapanthenol or a pharmaceutically acceptable salt thereof is present in the aqueous phase and mometasone and/or fluticasone or a pharmaceutically acceptable salt on any one of the foregoing being suspended therein.

6. The combination preparation of any one of claims 1 to 5 wherein one or more of the ingredients of the combination preparation according to the present invention are present in microencapsulated form.
